# EUROPÄISCHE PATENTSCHRIFT

(11) **EP 1 790 670 B1**
(45) Veröffentlichungstag und Bekanntmachung des Hinweises auf die Patenterteilung: **25.08.2010**
(21) Anmeldenummer: 07104121.4
(22) Anmeldetag: 22.01.2002
(51) Int. Cl.: C08F 10/02, C08K 5/07

(54) **Verwendung von Ketonen als Regler und als Lösemittel für Radikalstarter bei der Herstellung von Ethylenpolymerisaten im Hochdruckverfahren**
Use of ketones as chain transfer agent and as solvent for radical initiators in a high pressure polymerisation process for polyethylenes
Utilization de cétones comme agent de transfer de chaîne et comme solvent pour des initiateurs dans un procédé de production de polymères d'éthylène sous haute préssion

(30) Priorität: 23.01.2001 DE 10102937
(43) Veröffentlichungstag der Anmeldung: 30.05.2007
(62) Teilanmeldung aus: 02714098.7
(73) Patentinhaber: Basell Polyolefine GmbH, 50389 Wesseling (DE)
(72) Erfinder: Wittkowski, Lars, 67056, Ludwigshafen (DE); Deckers, Andreas, 55234, Flomborn (DE); Weber, Wilhelm, 67435, Neustadt (DE); Dröge, Thomas, Morris Town 07960 New Jersey (US); Gonioukh, Andrei, 50374, Erftstadt (DE); Mähling, Frank-Olaf, 68165, Mannheim (DE)

(56) Entgegenhaltungen:
- EP-A- 0 449 092
- EP-A1- 0 928 797
- WO-A-02/051885
- US-A- 3 963 690

## Beschreibung

Die vorliegende Erfindung betrifft die Verwendung eines oder mehrerer Ketone in einem Hochdruckverfahren mit einem oder mehreren Radikalstartern zur Herstellung von höhermolekularem LDPE als Regler zur Einstellung des Molekulargewichts des höhermolekularem LDPEs und als Lösemittel für den oder die Radikalstarter.

Ethylenpolymerisate lassen sich nach verschiedenen Verfahren herstellen, die sich grob in Niederdruckverfahren, durchgeführt bei 20 bis 100 bar und Temperaturen bis 110°C, und Hochdruckverfahren, durchgeführt bei 500 bis 4000 bar und Temperaturen von 150°C und höher verwendet. Das Hochdruckverfahren ist ein radikalisches Polymerisationsverfahren, das im Allgemeinen ohne Katalysator auskommt (vgl. beispielsweise: Ullmann's Enyclopädie der technischen Chemie, 4. Auflage, Stichworte: Wachse, Bd. 24, S. 36 ff., Thieme Verlag Stuttgart, 1977). Zum Starten der radikalischen Kettenreaktion verwendet man meistens ein oder mehrere organische Peroxide, beispielsweise die Trigonox® oder Perkadox®-Marken der Akzo Nobel, oder aber Luft bzw. Luftsauerstoff. Das Verfahren wird generell in Hochdruckautoklaven oder in Rohrreaktoren durchgeführt. Hochdruckautoklaven sind in sogenannten gedrungenen oder langgestreckten Ausführungsformen bekannt. Die vielfach verwendeten Rohrreaktoren (Ullmanns Encyclopädie der technischen Chemie, Band 19, S. 169 und S. 173 ff (1980), Verlag Chemie Weinheim, Deerfield Beach, Basel sowie Ullmann's Encyclopädie der technischen Chemie, 4. Auflage, Stichworte: Wachse, Bd. 24, S. 36 ff., Thieme Verlag Stuttgart, 1977) zeichnen sich durch einfache Handhabung und geringen Wartungsaufwand aus.

Zur Einstellung des geeigneten Molekulargewichts verwendet man als Molekulargewichtsregler oder kurz Regler bezeichnete Substanzen. Bei der Verwendung eines Stoffes als Regler ist zu beachten, dass er hinreichend effizient ist, weil die Dosierung sehr großer Mengen an Reglern unwirtschaftlich ist.

Ein häufig verwendeter Regler ist Wasserstoff, der aber bei der Verwendung von Luft oder Luftsauerstoff als Radikalstarter in radikalischen Polymerisationsverfahren zur Bildung von Knallgas führen kann und deshalb aus sicherheitstechnischen Gründen Bedenken hervorruft.

Gasförmige Regler wie die häufig verwendeten Alkane Ethan und Propan erfordern ebenfalls strenge Sicherheitsregeln.

Als besonders vorteilhafte Regler sind Ketone bekannt, weil sie sich zur Herstellung ausgezeichneter Produkte mit vorteilhaften organoleptischen Eigenschaften eignen, wie beispielsweise in DE-A 100 64 752, DE-A 100 64 799 und DE-A 100 64 800 gezeigt wurde. Aus Kostengründen wird man jedoch bestrebt sein, den Verbrauch an Ketonen so niedrig wie möglich zu halten.

Die Verwendung von Ketonen als Molekulargewichtsregler bei der Herstellung von hochmolekularem LDPE ist ebenfalls bekannt. In EP-A 0 928 797 wird ein Verfahren unter Verwendung von Methylethylketon als Regler vorgeschlagen, mit Hilfe dessen ein LDPE hergestellt wird, das sich für Extrusionsprodukte eignet, beispielsweise für Folien mit gutem Durchstichwiderstand. Der Verbrauch an Regler ist jedoch sehr hoch, was wirtschaftlich von Nachteil ist. Wollte man nach dem in EP-A 0 928 797 beschriebenen Verfahren Wachse herstellen, wäre der Verbrauch an Regler noch wesentlich höher.

In DE-A 1 908 964 wird ein Verfahren offengelegt, durch das sich Ethylenhomopolymerisate im Hochdruckverfahren herstellen lassen. Kennzeichnend an dem beschriebenen Verfahren ist die Verwendung eines peroxidischen Radikalstarters, zweckmässigerweise gelöst in einem inerten Lösemittel (S. 4) in der ersten Reaktionszone und Luft in der zweiten. Als Regler werden Propionaldehyd oder Methylethylketon empfohlen. In diesem Verfahren werden Ethylen, Radikalstarter und Regler gleichzeitig in der ersten Reaktionszone in den Reaktor dosiert, in einer zweiten Reaktionszone werden Luft und ein weiterer Regler dosiert. Das beschrieben Verfahren erfordert einen erheblichen logistischen und verfahrenstechnischen Aufwand.

In US 3,334,081 wird ein Hochdruck-Polymerisationsverfahren mit erhöhtem Umsatz beschrieben, das auf der Einspeisung von Ethylen an mindestens zwei verschiedenen Stellen des Reaktors beruht. Als Radikalstarter werden eine Vielzahl von organischen Peroxiden und als Regler eine Vielzahl organischer Verbindungen, bevorzugt Ketone wie beispielsweise Methylethylketon, empfohlen. Nachteilig an dem beschriebenen Verfahren ist jedoch der hohe Investitionsaufwand, der auf den zahlreichen Dosierstellen beruht, die alle extrem druckstabil und dicht ausgelegt werden müssen. Dadurch wird der Investitionsbedarf für eine Polymerisationsanlage sehr hoch.

WO 02/051885 offenbart ein Verfahren zur Herstellung von Ethylenpolymeren in einem Hochdruckverfahren, bei dem Sauerstoff als Initiator der Polymerisation eingesetzt wird und aliphatische Ketone als Molmassenregler verwendet werden.

US 3,963,690 beschreibt ein Verfahren zur Polymerisation von Ethylen unter Hochdruckbedingungen mit einem speziellen gerührten Autoklavenreaktor. Als Radikalstarter können Peroxide zum Einsatz kommen, die mit Löse- oder Verdünnungsmitteln gemischt sein können.

Es bestand also die Aufgabe, ein Verfahren bereitzustellen, durch das Polyethylen, beispielsweise hochmolekulares Polyethylen, im Hochdruckverfahren hergestellt werden kann und bei dem sowohl der Verbrauch an Prozesslösemitteln und Reglern, insbesondere an Ketonen als Regler, als auch Anzahl und Menge an verschiedenen Prozesschemikalien möglichst gering ist.

Die Aufgabe lässt sich überraschend so lösen, dass man ein oder mehrere Ketone der allgemeinen Formel I 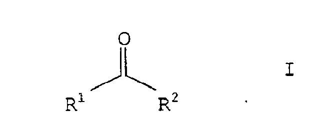 bei dem R¹ und R² gleich oder verschieden sind und ausgewählt werden aus C₁-C₆-Alkyl oder C₃-C₁₂-Cycloalkyl, wobei R¹ und R² auch kovalent miteinander unter Bildung eines 4- bis 13-gliedrigen Rings verknüpft sein können,
in einem Hochdruckverfahren mit einem oder mehreren Radikalstartern zur Herstellung von höhermolekularem LDPE mit einem Molekulargewicht M_{w} höher als 40.000 g
als Regler zur Einstellung des Molekulargewichts des höhermolekularen LDPEs und als Lösemittel für den oder die Radikalstarter verwendet,
wobei man den oder die Radikalstarter in dem Keton oder in den Ketonen der allgemeinen Formel I löst, komprimiert, an einer oder mehreren Stellen in den Polymerisationsreaktor dosiert und anschließend bei 140 bis 350°C und 400 bis 4500 bar polymerisiert.

Als Regler verwendet man ein oder mehrere aliphatische, cycloaliphatische oder alicyclische Ketone der allgemeinen Formel I 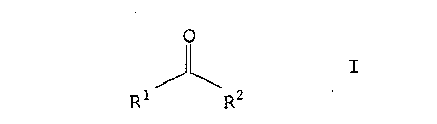 Dabei sind die Reste R¹ und R² gleich oder verschieden und ausgewählt aus
- C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und Lert.-Butyl;
- C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;

In einer besonderen Ausführungsform sind die Reste R¹ und R² miteinander unter Bildung eines 4- bis 13-gliedrigen Rings kovalent verbunden. So können R¹ und R² beispielsweise gemeinsam sein:
- (CH₂)₄-, -(CH₂)₅-, -(CH₂)₆, -(CH₂)₇-, -CH(CH₃)-CH₂-CH₂-CH(CH₃)-oder
- CH(CH₃)-CH₂-CH₂-CH₂-CH(CH₃)-.

Bevorzugte Beispiele sind Aceton, Methylethylketon "MEK", Methylisobutylketon "MIBK", 2-Pentanon, 3-Pentanon oder Cyclopentanon, Cyclohexanon oder Cycloheptanon. Besonders bevorzugte Beispiele sind Aceton, Methylethylketon, Methylisobutylketon und Cyclohexanon; ganz besonders bevorzugt sind Methylethylketon und Cyclohexanon.

Als Radikalstarter verwendet man ein oder mehrere Peroxide, ausgewählt aus den kommerziell erhältlichen Substanzen
- Didecanoylperoxid, 2,5-Dimethyl-2,5-di(2-ethylhexanoylperoxy)hexan, tert.-Amylperoxy-2-ethylhexanoat, Dibenzoylperoxid, tert.-Butylperoxy-2-ethylhexanoat, tert.Butylperoxydiethylacetat, tert.Butylperoxydiethylisobutyrat, 1,4-Di(tert.-butylperoxycarbo)-cyclohexan als Isomerengemisch, tert.-Butylperisononanoat 1,1-Di-(tert.-butylperoxy)-3,3,5-trimethylcyclohexan, 1,1-Di-(tert.-butylperoxy)-cyclohexan, Methyl-isobutylketonperoxid, tert.-Butylperoxyisopropylcarbonat, 2,2-Di-tert.-butylperoxy)butan oder tert.-Butylperoxyacetat;
- tert.-Butylperoxybenzoat, Di-tert.-amylperoxid, Dicumylperoxid, die isomeren Di-(tert.-butylperoxyisopropyl)benzole, 2,5-Dimethyl-2,5-di-tert.-butylperoxyhexan, tert.-Butylcumylperoxid, 2,5-Dimethyl-2,5-di(tert.-butylperoxy)-hex-3-in, Ditert.-butylperoxid, 1,3-Diisopropylmonohydroperoxid, Cumolhydroperoxid oder tert.-Butylhydroperoxid; oder
- dimere oder trimere Ketonperoxide der allgemeinen Formel II a
   bis II c.

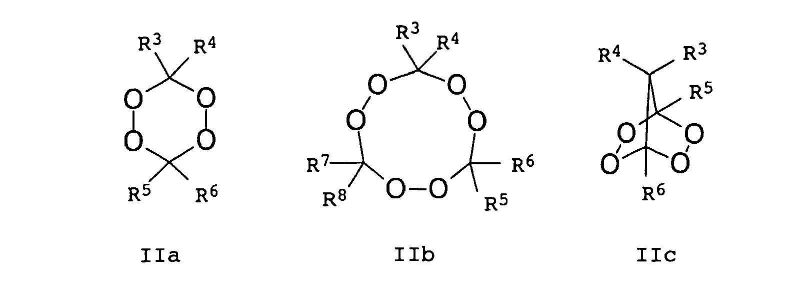

Dabei sind die Reste R³ bis R⁸ gleich oder verschieden und ausgewählt aus
- C₁-C₈-Alkyl wie Methyl, Ethyl, n-Propyl, iso-Propyl, n-Butyl, sec.-Butyl, iso-Butyl, tert.-Butyl, n-Pentyl, sec.-Pentyl, iso-Pentyl, n-Hexyl, n-Heptyl, n-Octyl; bevorzugt lineares C₁-C₆-Alkyl wie Methyl, Ethyl, n-Propyl, n-Butyl, iso-Butyl, n-Pentyl, iso-Pentyl, n-Hexyl, iso-Hexyl, sec.-Hexyl, besonders bevorzugt lineares C₁-C₄-Alkyl wie Methyl, Ethyl, n-Propyl oder n-Butyl, ganz besonders bevorzugt ist Ethyl;
- C₆-C₁₄-Aryl wie Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
   Peroxide der allgemeinen Formeln II a bis II c sowie Verfahren zu ihrer Herstellung sind aus EP-A 0 813 550 bekannt.

Ein oder mehrere Peroxide, die in Substanz oder in Lösung kommerziell erworben werden können, werden in einem oder mehreren Ketonen der allgemeinen Formel I gelöst. Optional können weitere gängige Lösemittel zur Phlegmatisierung der Radikalstarter zugegeben werden, beispielsweise Wasser, Toluol, Chlorbenzol, Weißöl, Silikonöle oder vorzugsweise aliphatische Lösemittel wie Isododekan, Exxsol® oder Isopar Fluids® der Firma Exxon. Vorzugsweise wird jedoch auf weitere Lösemittel verzichtet.

Als Konzentration des oder der Radikalstarter hat sich 5 bis 35 Gew.-%, bevorzugt 10 bis 30 und besonders bevorzugt 15 bis 25 Gew.-% bewährt.

Wenn der Radikalstarter bei Raumtemperatur bereits in flüssiger Form vorliegt, so ist auch das Verdünnen des Radikalstarters mit einem oder mehreren Ketonen eine Ausführungsform der vorliegenden Erfindung. Dabei ist es unerheblich, ob das Vorliegen der flüssigen Form dadurch bedingt ist, dass der Initiator einen Schmelzpunkt unterhalb Raumtemperatur hat oder ob er aus Sicherheitsgründen nur als stark konzentrierte, phlegmatisierte Lösung in einem Kohlenwasserstoff in den Handel kommt.

Anschließend wird die Lösung des oder der Radikalstarter auf an sich bekannte Weise komprimiert, an einer oder mehreren Stellen in den Polymerisationsreaktor dosiert und danach polymerisiert. Das Komprimieren erfolgt unter Kühlung oder bevorzugt bei Raumtemperatur. Trotz der Tatsache, dass sich die eingesetzten Peroxide leicht zersetzen, ist ein Mischen und Komprimieren gefahrlos möglich.

Auch ist es möglich, die hergestellten Lösungen aus Keton oder Ketonen, einem oder mehreren Radikalstartern sowie optional dem Lösemittel längere Zeit aufzubewahren oder, falls notwendig, durch längere Rohrleitungen hindurch zu pumpen, ohne dass Zersetzungserscheinungen der Peroxide beobachtet werden müssen.

Bei der erfindungsgemäßen Verwendung erfolgt die Polymerisation oder Copolymerisation üblicherweise bei Drücken von 400 bis 4500 bar, bevorzugt von 500 bis 4000 bar und besonders bevorzugt 1000 bis 3500 bar.

Die Polymerisationstemperatur beträgt 140 bis 350°C, bevorzugt sind 200 bis 320°C.

Die durch die beschriebene Verwendung erhältlichen Polyethylene haben eine Dichte von 0,900 bis 0,955 g/cm³, bevorzugt von 0,910 bis 0,945 g/cm³ und besonders bevorzugt von 0,915 bis 0,940 g/cm³, gemessen bei 23°C. Die erfindungsgemäße Verwendung ist zur Herstellung von höhermolekularem LDPE mit einem Molekulargewicht M_{w}, das höher ist als 40.000 g, bevorzugt höher als 80.000 g und besonders bevorzugt höher als 120.000 g gut geeignet. Die Molekulargewichtsverteilung des hergestellten höhermolekularen LDPE liegt im Bereich von 2 bis 20. Die Schmelzpunkte liegen im Bereich von 80 bis 135°C, bevorzugt 100 bis 125°C.

Es lassen sich auch Copolymerisate mit Ethylen herstellen, wobei prinzipiell alle radikalisch mit Ethylen copolymerisierbaren Olefine als Comonomere geeignet sind. Bevorzugt sind
- 1-Olefine wie Propylen, 1-Buten, 1-Penten, 1-Hexen, 1-Octen und 1-Decen,
- Acrylate wie Acrylsäure, Acrylsäuremethylester, Acrylsäureethylester, Acrylsäure-n-butylester, Acrylsäure-2-ethylhexylester oder Acrylsäure-tert.-butylester;
- Methacrylsäure, Methacrylsäuremethylester, Methacrylsäureethylester, Methacrylsäure-n-butylester oder Methacrylsäuretert.-butylester;
- Vinylcarboxylate, wobei Vinylacetat besonders bevorzugt ist,
- Ungesättigte Dicarbonsäuren, besonders bevorzugt ist Maleinsäure,
- ungesättigte Dicarbonsäurederivate, besonders bevorzugt sind Maleinsäureanhydrid und Maleinsäurealkylimide wie beispielsweise Maleinsäuremethylimid.

Der Comonomeranteil beträgt maximal 50 mol-%, bevorzugt maximal 20 mol-%.

Das erfindungsgemäße Verfahren erlaubt es, bei deutlicher Einsparung an Regler, Radikalstarter und Lösemittel zu polymerisieren. Das bedeutet einerseits eine Einsparung an Einsatzstoffen; andererseits müssen beispielsweise die Lösemittelreste oder Zersetzungsproduktc des Radikalstarters nach der Reaktion nicht aus dem Produkt entfernt werden. Weiterhin wird durch die verminderte Zahl an Einsatzstoffen der logistische Aufwand in der Produktion und auch die Sicherheit des Verfahrens wesentlich erhöht.

Außerdem wird beim erfindungsgemäßen Verfahren ein höherer Umsatz beobachtet, als wenn man wie im Stand der Technik eine Lösung des Radikalstarters und den Regler getrennt dosiert.

Die durch die erfindungsgemäße Verwendung hergestellten höhermolekularen Ethylenpolymere zeigen sehr gute organoleptische Eigenschaften, insbesondere sind sie Geruchs- und Geschmacks-neutral. Die Messung organoleptischer Eigenschaften lässt sich apparativ, beispielsweise durch Gaschromatographie oder Differentialthermogravimetrie, bestimmen, wobei durch getrennte oder hintereinandergeschaltete Messapparaturen die Menge und die Art der entweichenden flüchtigen Verbindungen ermittelt wird. Von hoher Signifikanz sind die Tests durch Probandenteams.

Die durch die erfindungsgemäße Verwendung erhältlichen Ethylenpolymerisate mit einem Molekulargewicht M_{w} über 40.000 g eignen sich beispielsweise ausgezeichnet zur Herstellung von Beuteln für Lebensmittelverpackunqen.

Die Erfindung wird durch Arbeitsbeispiele erläutert.

Ethylen wurde in einem Hochdruckautoklaven polymerisiert, wie er in der Literatur beschrieben wird (M. Buback et al., Chem. Ing. Tech. 1994, 66, 510.)

Für die Versuche 1 und 2 wurde jeweils eine Lösung aus Radikalstarter in Methylethylketon angesetzt. Die Dosierrate an äquimolarem Gemisch der Peroxide tert.-Butylperoxypivalat und tert.-Butylperoxy-3,3,3-trimethylhexanoat als Radikalstarter in Methylethylketon sind in Tabelle 1 angegeben. Anschließend wurden Monomer oder Monomerengemisch durch eine Dosierleitung und die Lösung von Radikalstarter in Methylethylketon durch eine zweite Dosierleitung unter dem Reaktionsdruck von 1700 bar eingespeist. Es wurde bei einer Temperatur von 260°C polymerisiert.

Für die Vergleichsversuche V1-V3 wurde jeweils ein Gemisch aus Isododekan und den Radikalstartern angesetzt. Die Dosierrate an Isododekan und Radikalstarter (Gemisch aus tert.-Butylperoxypivalat und tert.-Butylperoxy-3,3,3-trimethylhexanoat im Molverhältnis 1,7 : 1) sind ebenfalls in Tabelle 1 angegeben. Anschließend wurden Monomer oder Monomerengemisch durch eine Dosierleitung, das Gemisch aus Isododekan und Radikalstarter durch eine zweite Dosierleitung und der Regler Methylethylketon durch eine dritte Dosierleitung unter dem Reaktionsdruck von 1700 bar eingespeist. Es wurde wiederum bei einer Temperatur von 260°C polymerisiert.

**Tabelle 1: Polymerisationsbedingungen der Beispiele 1 - 2 und der Vergleichsbeispiele V1 - V3.**

| Nr. | MEK | i-C₁₂ | Ethylen | Peroxid | PE | h | Umsatz |
|---|---|---|---|---|---|---|---|
| | [l/h] | [l/h] | [kg/h] | mol/h | kg/h | mm²/s | % |
| 1 | 1,93 | - | 9,9 | 0,010 | 2,8 | 300 | 28,3 |
| 2 | 1,01 | - | 10,1 | 0,012 | 2,7 | 1720 | 26,7 |
| V1 | 1,17 | 0,64 | 11,8 | 0,013 | 2,8 | 750 | 23,7 |
| V2 | 0,90 | 0,54 | 11,6 | 0,011 | 2,0 | 1500 | 17,2 |
| V3 | 0,75 | 0,57 | 12,0 | 0,011 | 2,5 | 3100 | 20,8 |

Verwendete Abkürzungen: MEK Methylethylketon, i-C₁₂: Isododekan; PE: Polyethylen, Ausbeute.

Polymerisationen bei 220°C:
Für die Versuche 3 und 4 wurde jeweils eine Lösung aus Methylethylketon und Radikalstarter angesetzt. Die Dosierrate an Methylethylketon und tert.-Butylperoxypivalat als Radikalstarter sind in Tabelle 2 angegeben. Anschließend wurden Monomer oder Monomerengemisch durch eine Dosierleitung sowie die Lösung des tert.-Butylperoxypivalat in Methylethylketon durch eine zweite Dosierleitung unter dem Reaktionsdruck von 1700 bar eingespeist. Es wurde bei einer Temperatur von 220°C polymerisiert.
Für die Vergleichsversuche V4 und V5 wurde jeweils ein Gemisch aus Isododekan und dem Radikalstarter angesetzt. Die Dosierrate an Isododekan und tert.-Butylperoxypivalat als Radikalstarter sind in Tabelle 2 angegeben. Anschließend wurden Monomer oder Monomerengemisch durch eine Dosierleitung, das Gemisch aus Isododekan und Radikalstarter durch eine zweite Dosierleitung und der Regler Methylethylketon durch eine dritte Dosierleitung unter dem Reaktionsdruck von 1700 bar eingespeist. Es wurde wiederum bei einer Temperatur von 220°C polymerisiert.

**Tabelle 2: Polymerisationsbedingungen der Beispiele 3 - 4 und der Vergleichsbeispiele V4 - V5.**

| Nr. | MEK | i-C₁₂ | Ethylen | Peroxid | PE | h | Umsatz |
|---|---|---|---|---|---|---|---|
| | [l/h] | [l/h] | [kg/h] | mol/h | kg/h | mm²/s | % |
| 3 | 1,98 | - | 10,2 | 0,012 | 1,9 | 460 | 18,6 |
| 4 | 1,97 | - | 10,3 | 0,012 | 1,8 | 500 | 17,5 |
| V4 | 1,65 | 0,50 | 12,2 | 0,010 | 2,0 | 750 | 16,4 |
| V5 | 2,50 | 0,38 | 10,7 | 0,011 | 1,7 | 200 | 15,9 |

Verwendete Abkürzungen: MEK Methylethylketon, i-C₁₂: Isododekan; PE: Polyethylen.

## Patentansprüche

1. Verwendung eines oder mehrerer Ketone der allgemeinen Formel I 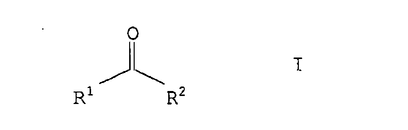 bei dem R¹ und R² gleich oder verschieden sind und ausgewählt werden aus C₁-C₆-Alkyl oder C₃-C₁₂-Cycloalkyl, wobei R¹ und R² auch kovalent miteinander unter Bildung eines 4- bis 13-gliedrigen Rings verknüpft sein können,
in einem Hochdruckverfahren mit einem oder mehreren Radikalstartern zur Herstellung von höhermolekularem LDPE mit einem Molekulargewicht M_{w} höher als 40.000 g
als Regler zur Einstellung des Molekulargewichts des höhermolekularen LDPEs und als Lösemittel für den oder die Radikalstarter,
wobei man den oder die Radikalstarter in dem Keton oder in den Ketonen der allgemeinen Formel I löst, komprimiert,
an einer oder mehreren Stellen in den Polymerisationsreaktor dosiert und anschließend bei 140 bis 350°C und 400 bis 4500 bar polymerisiert.

2. Verwendung von Methylethylketon nach Anspruch 1 als Regler zur Einstellung des Molekulargewichts des höhermolekularem LDPEs und als Lösemittel für den oder die Radikalstarter.

3. Verwendung nach Anspruch 1 oder 2, wobei als Radikalstarter ein oder mehrere Peroxide eingesetzt werden.

## Claims

1. Use of one or more ketones of the general formula I 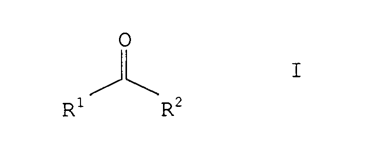 in which R¹ and R² are identical or different and are selected from C₁-C₆ alkyl or C₃-C₁₂ cycloalkyl, wherein R¹ and R² may also be covalently linked with one another to form 4- to 13-membered ring,
in a high pressure method with one or more free-radical initiators for producing relatively high molecular weight LDPE with a molecular weight M_{w} of greater than 40,000 g
as a chain-transfer agent for establishing the molecular weight of the relatively high molecular weight LDPE and as a solvent for the free-radical initiator(s),
wherein the free-radical initiator(s) is/are dissolved in the ketone(s) of the general formula I, compressed, dispensed at one or more locations into the polymerisation reactor and then polymerised at 140 to 350°C and 400 to 4500 bar.

2. Use of methyl ethyl ketone according to claim 1 as a chain-transfer agent for establishing the molecular weight of the relatively high molecular weight LDPE and as a solvent for the free-radical initiator(s).

3. Use according to claim 1 or claim 2, wherein one or more peroxides is/are used as the free-radical initiator.

## Revendications

1. Utilisation d'une ou de plusieurs cétones de formule générale
I 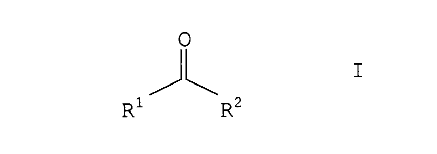 où R¹ et R² sont identiques ou différents et peuvent être choisis parmi C₁-C₆-alkyle ou C₃-C₁₂-cycloalkyle, R¹ et R² pouvant également être liés par covalence l'un à l'autre, avec formation d'un cycle de 4 à 13 chaînons,
dans un procédé haute pression avec un ou plusieurs initiateurs de radicaux pour la préparation de LDPE de haut poids moléculaire présentant un poids moléculaire M_{w} supérieur à 40 000 g
comme agent de régulation pour le réglage du poids moléculaire du LDPE de haut poids moléculaire et comme solvant pour le ou les initiateurs de radicaux,
où on dissout le ou les initiateurs de radicaux dans la ou les cétones de formule générale I, on comprime, on dose en un ou plusieurs endroits dans le réacteur de polymérisation, puis on polymérise à 140 jusqu'à 350°C et à 400 jusqu'à 4500 bars.

2. Utilisation de méthyléthylcétone selon la revendication 1 comme agent de régulation pour le réglage du poids moléculaire du LDPE de haut poids moléculaire et comme solvant pour le ou les initiateurs de radicaux.

3. Utilisation selon la revendication 1 ou 2, où on utilise comme initiateur de radicaux un ou plusieurs peroxydes.
